# FASCICULE DE BREVET EUROPEEN

(11) **EP 1 435 830 B1**
(45) Date de publication et mention de la délivrance du brevet: **03.12.2008**
(21) Numéro de dépôt: 03735778.7
(22) Date de dépôt: 14.03.2003
(51) Int. Cl.: A61B 1/267, A61B 19/02, B65D 75/38

(54) **ENSEMBLE COMPRENANT UNE LAME DE LARYNGOSCOPE ET UNE GAINE POUR CONTENIR ET MANIPULER LA LAME A USAGE UNIQUE**
KOMBINATION AUS LARYNGOSKOP-KLINGE UND HÜLLE FÜR DIE AUFNAHME UND MANIPULATION DER KLINGE ZUM EINMALGEBRAUCH
COMBINATION COMPRISING A LARYNGOSCOPE BLADE AND A SHEATH FOR CONTAINING AND MANIPULATING OF THE SINGLE-USE BLADE

(30) Priorité: 15.03.2002 FR 0203227
(43) Date de publication de la demande: 14.07.2004
(73) Titulaire: VYGON, F-95440 Ecouen (FR)
(72) Inventeur: DALLE, Valéry, F-60270 Gouvieux (FR); CARREZ, Jean-Luc, F-95440 Ecouen (FR); BROUILLON, André, F-60140 Rosoy (FR)
(74) Mandataire: Texier, Christian
(86) Numéro de dépôt international: PCT/FR2003/000822
(87) Numéro de publication internationale: WO 2003/077736

(56) Documents cités:
- FR-A- 1 459 949
- US-A- 2 902 146
- US-A- 2 949 181
- US-A- 5 347 995

## Description

L'invention concerne un ensemble comprenant une lame de laryngoscope et une gaine pour contenir et manipuler la lame de laryngoscope à usage unique destinée à être fixée de façon détachable sur un manche de laryngoscope.

On connaît de nombreux exemples d'une lame de laryngoscope qui présente une extrémité de fixation adaptée pour le montage et l'accrochage de la lame sur une extrémité de réception adaptée du manche : EP 0 110 333, EP 0 169 497, GB 2 191 949 et autres.

Habituellement, la lame de laryngoscope est conditionnée de façon stérile dans un emballage d'où la lame est extraite pour être fixée sur un manche en vue de son utilisation.

La publication US 5 347 995 expose la difficulté de stériliser une lame de laryngoscope et préconise l'utilisation d'une gaine stérile qui présente une extrémité ouverte et une extrémité fermée, et qui est apte à contenir la lame et à recouvrir en partie le manche du laryngoscope. L'utilisateur reçoit la gaine vide et emballée dans un emballage de protection. Pour utiliser un laryngoscope, l'utilisateur fixe la lame sur le manche, ouvre l'emballage de la gaine, introduit la lame dans l'extrémité ouverte de la gaine stérile jusqu'à ce que la lame soit contenue dans la gaine et recouvre l'extrémité du manche, et il fixe la gaine sur cette extrémité ; l'utilisateur élimine alors l'emballage de protection et le laryngoscope est prêt à l'emploi, la gaine restant en place sur la lame pour assurer la stérilité de la lame.

Cette gaine qui doit être jetée après usage (alors que la lame est destinée à être réutilisée), est destinée à protéger le patient d'un contact avec une lame non stérile mais ne protège pas l'opérateur d'un risque d'infection au contact du patient.

La présente invention a pour but de permettre à un praticien d'utiliser un laryngoscope sans risque d'infection et de contamination.

On y parvient selon la présente invention en plaçant la lame dans une gaine, entre une extrémité de gaine ouverte ou ouvrable et une extrémité de gaine opposée et fermée, la lame étant disposée dans la gaine en sorte que l'extrémité de fixation de la lame se trouve du côté de l'extrémité fermée de la gaine, cette gaine étant réalisée dans un film suffisamment mince, souple et résistant à la déchirure pour que l'opérateur puisse saisir la lame à travers la gaine pour fixer la lame sur son manche en sorte que l'extrémité fermée de la gaine reste interposée entre l'extrémité, de fixation de la lame et l'extrémité de réception du manche et pour que la gaine ainsi tenue puisse être retroussée sur le manche pour recouvrir le manche et dégager la lame, en vue de l'utilisation de la lame, et puisse être ensuite rabattue sur la lame pour recouvrir la lame après usage et séparée du manche avec la lame restant à l'intérieur de la gaine pour être mise au rebut avec la lame.

La gaine est par exemple réalisée dans un film fin, typiquement un film d'épaisseur inférieure à 100 micromètres, et apte à résister à une déchirure accidentelle lors du montage ou du démontage de la lame, malgré la finesse du film et le film est suffisamment transparent pour permettre une bonne transmission de la lumière.

Parmi les matériaux qui conviennent à cet effet, le polyuréthane est actuellement un matériau préféré.

On décrira ci-après un exemple de mise en oeuvre de l'invention, en référence aux figures du dessin joint sur lequel :

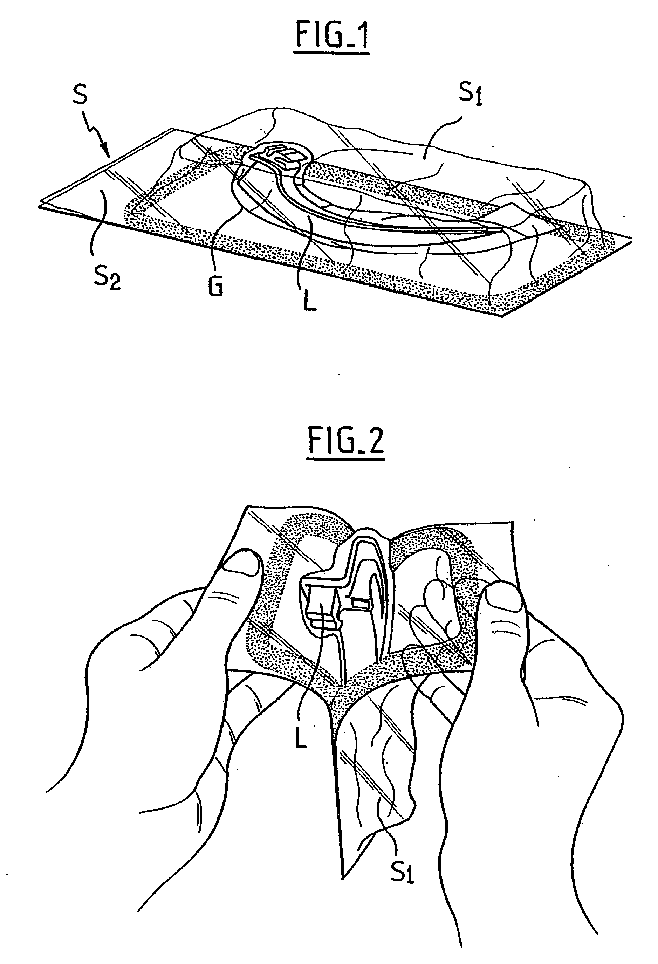

- la figure 1 est une perspective d'un ensemble constitué d'un sachet extérieur d'emballage fermé et contenant une gaine intérieure qui contient elle-même une lame de laryngoscope stérilisée ;
- la figure 2 est une perspective de l'ensemble de la figure 1 après ouverture du sachet extérieur, et
- les figures 3 à 10 montrent les différentes phases de mise en oeuvre d'une lame conditionnée dans une gaine selon l'invention.

Le sachet extérieur (S) est un sachet connu, par exemple un sachet pelable ou un blister, que l'on ouvre en « pelant ». Par exemple, ce sachet est constitué d'un film souple transparent (S1) fixé sur une feuille de support (S2), le sachet ayant une porosité appropriée pour permettre le passage d'un gaz ou d'un rayonnement au travers du sachet pour stériliser le contenu du sachet.

Le sachet étant ouvert, on en extrait la gaine intérieure qui contient intégralement une lame courbe de laryngoscope, par exemple une lame en matériau non métallique, tel que en résine thermoplastique.

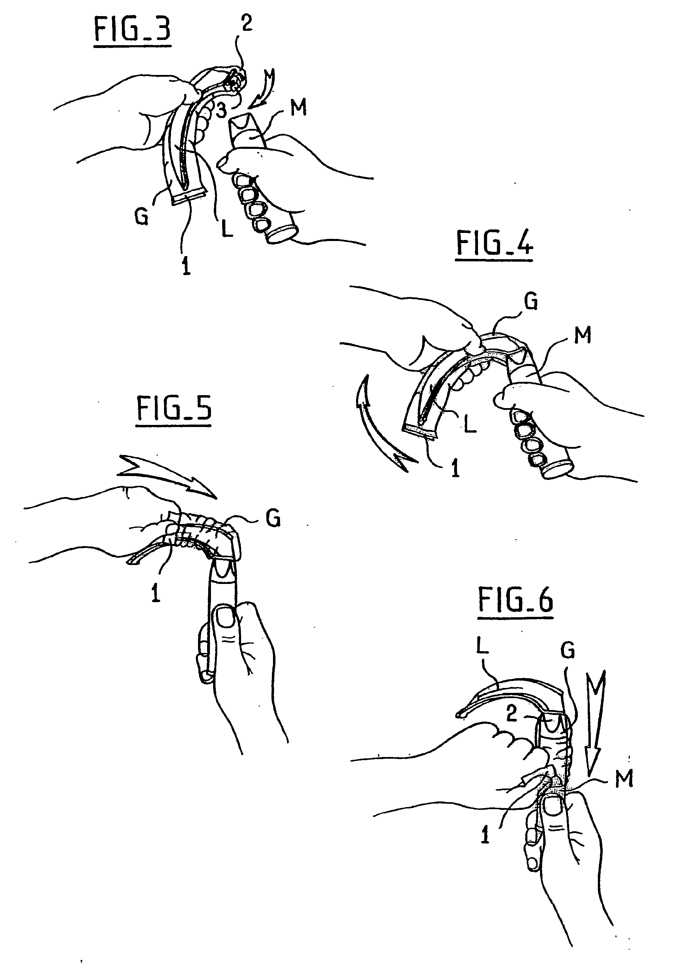

Sur la figure 3 du dessin joint, on a représenté cette gaine (G), par exemple un mince film transparent de polyuréthane contenant la lame (L) et tenue en main par l'opérateur.

Conformément à l'invention, cette gaine est un tube souple borgne à paroi très mince qui présente une extrémité (1) ouverte ou apte à être ouverte et une extrémité opposée (2) qui est fermée. La lame présente de façon en soi connue une extrémité (3) adaptée pour permettre le montage de la lame sur une extrémité d'un manche (M) que l'opérateur tient dans son autre main. La lame est placée dans la gaine en sorte que l'extrémité de montage (3) de la lame se trouve du côté de l'extrémité fermée (2) de la gaine.

Il n'est pas nécessaire de décrire plus en détails cette extrémité de la lame et l'extrémité correspondante du manche (M) qui sont bien connues en soi dans diverses réalisations et sur lesquelles ne portent pas l'invention.

Comme on le voit sur la figure 4, l'opérateur fixe ;la lame sur l'extrémité du manche sans que la gaine gêne cette fixation en raison de la très faible épaisseur de sa paroi.

Cette fixation étant faite, l'opérateur retrousse la gaine sur le manche comme on le voit sur les figures 5 et 6.

Le laryngoscope est alors prêt pour son utilisation!de façon en soi connue.

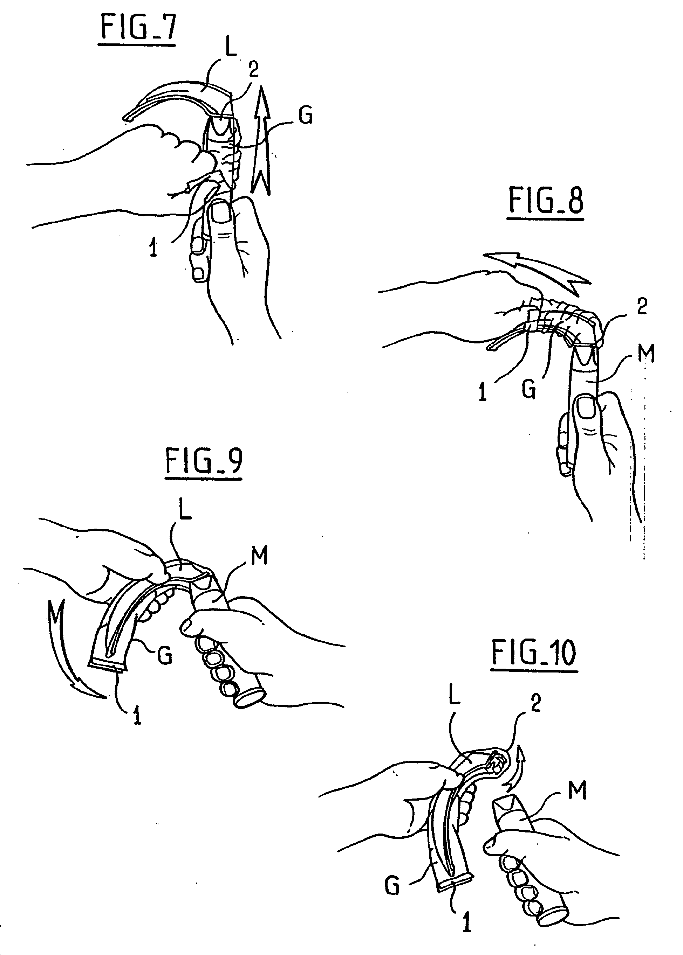

Après utilisation, la gaine (G) est retroussée en sens inverse (figures 7 et 8) pour revenir sur la lame après quoi la lame avec sa gaine qui la contient à nouveau peut être détachée du manche (figures 9 et 10) et mise au rebut. Avantageusement, l'extrémité ouverte de la gaine peut présenter des pattes qui facilitent le retroussage inverse, ce qui renforce la protection de l'opérateur vis-à-vis d'un risque de contact avec la lame.

L'invention n'est pas limitée à cet exemple de réalisation.

L'invention n'est pas limitée à un choix particulier des moyens qui permettent la fixation de la lame sur son manche pourvu que ces moyens ne soient pas susceptibles de déchirer la gaine. Dans l'exemple représenté sur les figures, ces moyens comprennent, de façon connue en soi, un talon formé à l'extrémité de fixation de la lame et une cavité apte à recevoir ce talon formée à l'extrémité de réception du manche, ledit talon présentant une partie de talon en forme de crochet et ladite cavité présentant un axe transversal avec lequel le crochet vient en prise lors du montage de la lame sur le manche ; en outre le talon et la cavité comportent des moyens de blocage de la lame montée sur le manche. Dans l'exemple représenté, ces moyens de blocage comportant une paroi élastique sur le talon de la lame et ut second axe transversal dans la cavité de l'extrémité du crochet, la paroi élastique venant en contact élastique avec ce second axe lors du montage de la lame sur le manche.

## Revendications

1. Ensemble comprenant une lame de laryngoscope et une gaine pour contenir et manipuler la lame, la lame présentant une extrémité de fixation (3) adaptée pour le montage de la lame sur une extrémité de réception d'un manche (M) de façon détachable, et la gaine contenant la lame de laryngoscope entre une extrémité de gaine (1) ouverte ou ouvrable et une extrémité de gaine (2) opposée et fermée, **caractérisé en ce que** la lame est disposée dans la gaine en sorte que l'extrémité de fixation (3) de la lame se trouve du côté de l'extrémité fermée (2) de la gaine, **en ce que** la gaine est réalisée dans un film suffisamment mince, souple et résistant à la déchirure pour que l'opérateur puisse saisir la lame à travers la gaine pour fixer la lame sur son manche en sorte que l'extrémité fermée de la gaine reste interposée entre l'extrémité de fixation de la lame et l'extrémité de réception du manche et pour que la gaine ainsi tenue puisse être retroussée sur le manche pour recouvrir le manche et dégager la lame, en vue de l'utilisation de la lame, et puisse être ensuite rabattue sur la lame pour recouvrir la lame après usage et séparée du manche avec la lame restant à l'intérieur de la gaine pour être mise au rebut avec la lame.

2. Ensemble selon la revendication 1 dont la gaine (G) est constituée dans un film d'épaisseur inférieure à 100 micromètres.

3. Ensemble selon la revendication 1 ou 2 dont la gaine est en polyuréthane.

4. Ensemble selon l'une des revendications 1 à 3 dont la gaine présente à son extrémité (1) ouverte ou ouvrante des pattes qui facilitent sa préhension.

5. Ensemble selon l'une des revendications 1 à 4, dont la lame est en matériau non métallique.

6. Ensemble selon l'une des revendications 1 à 5 et qui est placé à l'intérieur d'un sachet d'emballage (S) en film souple transparent de porosité appropriée pour permettre de stériliser le contenu du sachet au travers du sachet.

## Claims

1. An assembly comprising a laryngoscope blade and a sheath for containing and handling the blade, the blade presenting a fastening end (3) adapted for detachably mounting the blade on a receiving end of a handle (M), and the sheath containing the laryngoscope blade between an open or openable end of the sheath (1) and an opposite end of the sheath (2) that is closed, the assembly being **characterized in that** the blade is disposed in the sheath in such a manner that the fastening end (3) of the blade is adjacent to the closed end (2) of the sheath, and **in that** the sheath is made of a film that is sufficiently thin, flexible, and tear-resistant so that the operator can take hold of the blade through the sheath in order to fasten the blade on the handle with the closed end of the sheath remaining interposed between the fastening end of the blade and the receiving end of the handle, and so that the sheath as held in this way can be turned inside out onto the handle so as to cover the handle and uncover the blade in order to enable the blade to be used, and can subsequently be turned back over the blade so as to cover the blade after it has been used and separated from the handle with the blade remaining inside the sheath, which sheath is discarded together with the blade.

2. An assembly according to claim 1, in which the sheath (G) is constituted by a film of thickness less than 100 µm.

3. An assembly according to claim 1 or claim 2, in which the sheath is made of polyurethane.

4. An assembly according to any one of claims 1 to 3, in which the sheath presents tabs at its open or openable end (1) making it easier to take hold of the sheath.

5. An assembly according to any one of claims 1 to 4, in which the blade is of non-metallic material.

6. An assembly according to any one of claims 1 to 5, placed inside a packaging bag (S) of flexible transparent film of porosity suitable for enabling the content of the bag to be sterilized through the bag.

## Patentansprüche

1. Einheit, die einen Laryngoskop-Spatel und eine Schutzhülle zur Aufnahme und Handhabung des Spatels umfasst, wobei der Spatel ein Befestigungsende (3) hat, das für die abnehmbare Anbringung des Spatels an einem Aufnahmeende eines Haltegriffs (M) eingerichtet ist, wobei die Schutzhülle den Laryngoskop-Spatel zwischen einem Schutzhüllenende (1), das offen ist oder geöffnet werden kann, und einem entgegengesetzten und geschlossenen Schutzhüllenende (2) enthält, **dadurch gekennzeichnet, dass** der Spatel in der Schutzhülle derart angeordnet ist, dass das Befestigungsende (3) des Spatels sich auf der Seite des geschlossenen Endes (2) der Schutzhülle befindet, dadurch, dass die Schutzhülle aus einer Folie hergestellt ist, die ausreichend dünn, biegsam und reißfest ist, damit der Anwender den Spatel durch die Schutzhülle hindurch greifen kann, um den Spatel auf seinem Haltegriff derart zu befestigen, dass das geschlossene Ende der Schutzhülle zwischen dem Befestigungsende des Spatels und dem Aufnahmeende des Haltegriffs angeordnet verbleibt, und damit die so gehaltene Schutzhülle auf den Haltegriff umgestülpt werden kann, um für die Anwendung des Spatels den Haltegriff zu bedecken und den Spatel freizugeben, und anschließend auf den Spatel zurückgestülpt werden kann, um den Spatel nach dem Gebrauch wieder zu bedecken, und mit dem Spatel, der im Inneren der Schutzhülle verbleibt, vom Haltegriff getrennt werden kann, um zusammen mit dem Spatel in den Abfall gegeben zu werden.

2. Einheit nach Anspruch 1, deren Schutzhülle (G) aus einer Folie mit einer Dicke von weniger als 100 Mikrometer besteht.

3. Einheit nach Anspruch 1 oder 2, deren Schutzhülle aus Polyurethan besteht.

4. Einheit nach einem der Ansprüche 1 bis 3, deren Schutzhülle an ihrem Ende (1), das offen ist oder geöffnet werden kann, Laschen hat, die ihr Greifen vereinfachen.

5. Einheit nach einem der Ansprüche 1 bis 4, deren Spatel aus einem nichtmetallischen Material besteht.

6. Einheit nach einem der Ansprüche 1 bis 5, die im Inneren eines Verpackungsbeutels (S) platziert ist, der aus einer biegsamen und durchsichtigen Folie mit einer geeigneten Porosität besteht, um den Inhalt des Beutels durch den Beutel hindurch sterilisieren zu können.
